# EUROPEAN PATENT APPLICATION

(11) **EP 2 177 113 A1**
(43) Date of publication of application: **21.04.2010**
(21) Application number: 08166617.4
(22) Date of filing: 15.10.2008
(51) Int. Cl.: A23L 1/29, A23L 1/30, A23L 2/38, A23L 2/52

(54) **Improved liver glycocen synthesis**

(71) Applicant: Nestec S.A., 1800 Vevey (CH)
(72) Inventor: Decombaz, Jacques Edouard, 1806 St-Légier (CH); Jeukendrup, Asker, Birmingham, West Midlands B17 0TL (GB); Jentjens, Roy Leonardus Paulus Gerardus, 6071 JZ, Swalmen (NL)
(74) Representative: Elleby, Gudrun

(57) **Abstract**

This invention relates to a food product or beverage, in particular for the use in sports nutrition for replenishment of shortage in glycogen stored in the liver.
In particular the invention relates to a use of a food product or beverage comprising a combination of carbohydrates for replenishment of shortage in glycogen stored in the liver.

## Description

### Field of the Invention

This invention relates to a food product or beverage, in particular for the use in sports nutrition for replenishment of shortage in glycogen stored in the liver.

### Background of the invention

Athletes exercise with high frequency to improve skills and performance. However, an acute exercise is actually catabolic: the more intense and the longer the session, the higher the energy turnover leading to the depletion of energy stores in muscle and liver, to potential muscle damage, hypoglycaemia and an acute decrease in performance. A target of post-exercise recovery is restoration of energy stores.

Glycogen is the primary fuel supporting ATP (adenosine troposphere) homeostasis during moderate-to-intense exercise. The depletion of glycogen observed after prolonged exercise is associated with an accelerated rate of adenine nucleotide loss and fatigue. Resynthesis of glycogen during the recovery period after exercise is therefore important to the recovery of endurance exercise capacity.

However, depletion of glycogen from the liver may not be felt directly in the recovery. The effect of a depletion of the glycogen depot will nevertheless be felt by an athlete who tries to train or compete again before the depot of glycogen in the liver has been replenished, because low liver glycogen may lead faster to hypoglycaemia and fatigue.

Although hepatic glycogen synthesis may play an important role in the post exercise period, there is limited available evidence to suggest that liver glycogen resynthesis is a priority in the hours after exercise and that muscle glycogen synthesis is secondary.

One study investigated liver glycogen synthesis in the post-exercise period in healthy humans (Casey 2000) and found that 1 g/kg bm was sufficient to initiate liver glycogen synthesis without affecting muscle glycogen synthesis.

Fructose has been seen to have slightly different effects on liver glycogen metabolism than glucose, independent of the lower glycemic response with fructose. It was found that the bulk of an oral intravenous or oral fructose load is taken up by the liver and converted to glucose (Nilsson 1974).

Galactose is a monosaccharide and has the same chemical formula as glucose. Like glucose, galactose is actively absorbed by the small intestine through a sodium glucose transporter (SGLT1).

The liver is the major site of galactose uptake and metabolism in humans. In the liver, galactose can be converted to glucose and subsequently stored as glycogen or immediately released into the circulation.

US 6039987 (filed on 29.03.1999 by Strahl Robert Charles) discloses a product which comprises glucose, fructose and quinine. In particular this patent is concerned with a product to be taken during exercise and is aimed at preventing cramps. This patent is not concerned with product to be used in post exercise recovery.

Carbohydrate ingestion will not be stored in the liver during exercise and will therefore not increase the liver glycogen level, because the liver is continuously being tapped from during the exercise. In recovery after exercise, net storage occurs because glycogen breakdown has stopped and the depleted stores made greedy by the exercise retain the carbohydrates.

Whereas both muscle glycogen and liver glycogen are broken down during exercise, it is proper to liver (not muscle) glycogen to be used continuously, including when resting and during sleep. Therefore, liver glycogen may be in a depleted state while at the same time muscle glycogen is topped up.

Furthermore, carbohydrates absorbed from the food will first pass through the liver tissue, be captured in the liver according to need, and before the remaining fraction is circulated further to reach the muscles (Nuttall & Gannon, 2007).

There is a need for a better understanding of the effect of carbohydrate types on hepatic glycogen synthesis. Further there is a need for products that are directed to speeding up the replenishment of glycogen reserves in the liver after depletion of glycogen resulting from prolonged exercise to provide a faster recovery. In particular there is a need for products directed to the post exercise recovery. There is also a need for products for speeding up a person's replenishment of glycogen reserves in the liver when the liver glycogen reserves are low e.g. after a night's sleep.

The present invention seeks to address the above-described problems. The invention also aims at other objects and particularly the solution of other problems as will appear in the rest of the present description.

### Object and summary of the invention

The present invention relates to the use of a food product or beverage comprising a combination of carbohydrates for replenishment of shortage in glycogen stored in the liver.

In particular it concerns a food product or beverage comprising a combination of carbohydrates comprising
- glucose and/or glucose delivering carbohydrates, and
- fructose and/or fructose delivering carbohydrates. Preferably, the glucose delivering carbohydrate is maltodextrin.

The invention also concerns a use of a food product or beverage for replenishment of shortage in glycogen stored in the liver comprising a combination of carbohydrates comprising
- glucose and/or glucose delivering carbohydrates, and
- galactose and/or galactose delivering carbohydrates. Preferably, the glucose delivering carbohydrate is maltodextrin.

The ingestion of glucose with the aim of speeding up the recovery of glycogen stores in the body meets with difficulties in relation with a limitation (a ceiling) in the capacity for the intestinal absorption of glucose molecules. Because of osmotic water movements to the gut, intestinal intolerance to such a large osmotic glucose load is not uncommon. It has been found that the latter can be avoided by partly replacing glucose for maltodextrins (a glucose delivering carbohydrate polymer). Osmotic load in the gut is reduced, leading to a better digestive tolerance and to the possibility of providing the large amounts of carbohydrate that are necessary for a fast glycogen synthesis is made possible.

Carbohydrate-derived glucose is the major precursor for liver glycogen synthesis in man with normal diets, which usually contains 30-50% starch. Glycogen is entirely made of glucose units. Therefore it was thought that a carbohydrate blend including maltodextrin and glucose (which should go with an improved gastro-intestinal tolerance at high intake compared with equicaloric glucose) would be the ideal carbohydrate feeding source for fast glycogen recovery. As will be seen from the example below this was found not to be the case.

It was therefore surprisingly observed that both the carbohydrate blend with fructose and the carbohydrate blend with galactose were two-fold more effective in stimulating glycogen synthesis than the proposed maltodextrin+glucose blend.

Fructose is not well absorbed by man in isolation, however when it is ingested simultaneously with glucose, the intestinal absorption is believed to be facilitated. Fructose is a precursor of liver glycogen, but it requires to be first converted to 3-carbon compounds in the liver.

Further, it was found that ingestion of a blend or combination of glucose and/or maltodextrin with fructose offered an alternative product to the glucose based recovery products. In particular this carbohydrate combination showed particular efficient for liver glycogen replenishment.

Galactose is known to be well absorbed by man and it is a preferred precursor for liver glycogen. However, only small amounts of galactose are consumed in the normal diet of adults, which may be why galactose shares the same active intestinal transporter system as glucose. When galactose is ingested simultaneously with glucose, no improvement in total carbohydrate absorption would be expected. At best, a carbohydrate combination of glucose and/or maltodextrin with galactose would be considered to produce the same liver glycogen synthesis as the maltodextrin+glucose. According to the present invention, it has been demonstrated that this carbohydrate combination in a food or beverage product is two-fold more effective in stimulating glycogen synthesis than proposed maltodextrin+glucose combinations.

Similar to the carbohydrate blends with fructose, and the carbohydrate blends with galactose showed a surprisingly increased efficiency of replenishment. At the same time, the consumption of all three products (carbohydrate blends with glucose, carbohydrate blends with fructose, and carbohydrate blends with galactose) were found to be equally well tolerated as judged from the subjective gastrointestinal symptoms reported. There were therefore not found any negative side effects of the more efficient carbohydrate combinations.

In a further aspect, the invention relates to a post exercise food product or beverage for replenishment of shortage in glycogen stored in the liver comprising a combination of carbohydrates comprising
- glucose and/or glucose delivering carbohydrates, and
- fructose and/or fructose delivering carbohydrates,
in a ratio of 4:1 to 1:1, preferably from 3:1 to 2:1.

The invention also relates to a post exercise food product or beverage for replenishment of shortage in glycogen stored in the liver comprising a combination of carbohydrates comprising
- glucose and/or glucose delivering carbohydrates, and
- galactose and/or galactose delivering carbohydrates. in a ratio of 4:1 to 1:1, preferably from 3:1 to 2:1.

### Detailed Description of the Invention

In the present context the following meanings are given to the terms:

All references to percentages are percentages by weight (of dry matter) unless otherwise stated. The ratio mentioned below is also given in weight of the molecules. Thus, for glucose delivering carbohydrates, fructose delivering carbohydrates, and galactose delivering carbohydrates only the weight of the glucose molecules, fructose molecules or galactose molecules respectively counts toward the ratio.

In the present context, Post-exercise recovery may be defined as the process by which rest and nutrition restore the body back to pre-exercise equilibrium and to the ability to perform equally again. Recovery may last hours or days, depending on the metabolic demand of the previous exercise and the quality of the nutritional management during the recovery. However, when recovery has to be fast, special nutritional products that are well tolerated in large amount, efficiently absorbed and stored, have a distinct advantage which is what the present invention offers.

Glycogen is the storage form of carbohydrates in muscles and liver and is derived from food carbohydrates.

Glucose delivering carbohydrates are carbohydrates which when absorbed in the body (through the intestinal membrane) deliver glucose. Glucose delivering carbohydrates include sucrose, dextrin, maltodextrin, starch, glucose polymers, maltose, lactose, high fructose corn syrup or combinations thereof.

Fructose delivering carbohydrates are carbohydrates which when absorbed in the body deliver fructose. Fructose delivering carbohydrates include high fructose corn syrup and fructose polymer, honey, inulin etc. Fructose gets transformed to glucose only after it has reached the liver.

Galactose delivering carbohydrates are carbohydrates which when absorbed in the body deliver galactose. Galactose delivering carbohydrates may be lactose. Galactose gets transformed to glucose only after it has reached the liver.

In a preferred embodiment the invention is concerned with a use of a food product or beverage, and the food product or beverage as such, with a ratio of 4:1 to 1:1
between
1) glucose and glucose delivering carbohydrates and
2) fructose and fructose delivering carbohydrates. Preferably, the ratio is from 3:1 to 2:1.

These ratios have been found to be the most effective carbohydrate combinations for post-exercise liver glycogen synthesis. More preferably, the
- Glucose delivering carbohydrates need to contribute at least one third of the carbohydrate combination.
- Fructose molecules in the gut should be below half of the carbohydrates being fed because of potential absorption problems with high fructose loads. It has surprisingly been found that a food or beverage product with this combination of carbohydrates is particular effective for rapid post-exercise liver glycogen synthesis.

In another preferred embodiment the invention is concerned with a use of a food product or beverage, and the food product or beverage as such, with a ratio of 4:1 to 1:1 between
1) glucose and glucose delivering carbohydrates and
2) galactose and galactose delivering carbohydrates. Preferably, the ratio is from 3:1 to 2:1.

For this embodiment of the invention, these ratios have been found to be the most effective carbohydrate combinations for post-exercise liver glycogen synthesis. More preferably, the
- Glucose delivering carbohydrates need to contribute at least one third of the carbohydrate combination and
- Galactose molecules in the gut should be below half of the carbohydrates being fed because of potential saturation of the intestinal transporter it has in common with glucose.

Furthermore, fructose and galactose use distinct absorption routes and also follow different biochemical pathways compared with glucose. It is therefore believed that their effects might be additive or synergic. A combination of
1) glucose and glucose delivering carbohydrates and
2) fructose and fructose delivering carbohydrates and
3) galactose and galactose delivering carbohydrates for the use according to the invention may be preferred. For example, a ratio of 1:1:1 glucose:fructose:galactose combination may be advantageously used.

A recommended use according to the invention is an intake of the food product or beverage amount that corresponds to an ingestion of 60 to 90 grams of carbohydrate per hour, preferable from 70 to 90 grams per hour.

For the embodiments of the invention described above, it is particularly preferred that the food product or beverage has an energy density of 1450 to 1970 kj/100 gram based on dry matter.

In preferred embodiments of the invention, it is concerned with the use for increasing the speed of replenishment of shortage in glycogen stored in the liver. The speed of replenishment is obtained due to the special combination of the carbohydrates according to the invention. In particular, this is useful for in post-exercise recovery for replenishment of liver glycogen storage.

While the present invention is primarily intended for athletes, it is however, clear that it can be used by anybody in the need for liver glycogen replenishment. For example, it may be used for the post-sleep liver glycogen storage replenishment. A further preferred embodiment of the invention concerns a use in post-sleep recovery for replenishment of liver glycogen storage. For the athlete, a particular situation where an effective and fast liver glycogen replenishment without the hindrance of gastro-intestinal discomfort is a distinct advantage is when two intense exercises are scheduled on the same day with only a few hours rest in-between. A further preferred embodiment of the invention therefore concerns recovery nutrition following the first of such consecutive exercises.

### Brief description of the drawings

- Fig. 1: shows a design of the study giving a comparison of intake glucose, fructose, and galactose products.
- Fig. 2: shows an increase in recovery of the glycogen concentration in the liver over time from intake of products of the invention versus a reference product.

Although the present invention has been described with reference to preferred embodiments thereof, many modifications and alternations may be made by a person having ordinary skill in the art without departing from the scope of this invention which is defined by the appended claims.

Preferred product compositions for post exercise products according to the invention are given in Table 1:

**Table 1.**

| Ingredients | Range (% w/w dry matter) | Comments |
|---|---|---|
| Carbohydrate | 60-100 | |
| Glucosyl-oligomer | | 33-75 %carbohydrate |
| Glucose | | 0-75 %carbohydrate |
| Galactose | | 0-35 %carbohydrate |
| Fructose | | 0-35% carbohydrate |
| Fat | 0-10 | |
| Fibre | 0-10 | |
| Protein | 0-15 | |
| Leucine | | 0-15 %protein |
| Vitamins | 0-30 % RDA | |
| Sodium | 0-1 | |
| Potassium | 0-0.2 | |

Tables 2 to 4 show examples of recipes for a dry powder product to be dissolved in 100ml liquid, e.g. water.

**Table 2. Exemples of recipe**

| **Drink 1. Powder, 15g for preparing 100ml** | |
|---|---|
| Ingredient | Weight |
| Maltodextrin Glucidex DE-12 | 67 |
| Fructose | 33 |
| TOTAL | 100 |

**Table 3. Example of recipe**

| **Drink 2. Powder, 15g for preparing 100ml** | |
|---|---|
| Ingredient | Weight |
| Maltodextrin Glucidex DE-12 | 66 |
| Galactose | 33 |
| Sodium chloride | 1 |
| TOTAL | 100 |

**Table 4. Example of recipe**

| **Drink 3. Powder, 17g for preparing 100ml** | |
|---|---|
| Ingredient | Weight |
| Maltodextrin MD-40 | 45 |
| Glucose monohydrate | 5 |
| Fructose | 15 |
| Galactose | 20 |
| Whey protein | 4.9 |
| Sodium chloride | 1.1 |
| Citric acid | 4 |
| Potassium chloride | 1 |
| Magnesium tri-citrate | 1 |
| Vitamin B1 | 0.0008 |
| Vitamin B2 | 0.0009 |
| Vitamin B6 | 0.0010 |
| Flavour mix containing black tea | 3 |
| extract and fruit flavours | |
| TOTAL | 100 |

### EXAMPLE 1

### Subjects and Preliminary Testing

Ten healthy, male endurance trained volunteers (mainly bicycle couriers) were included in the study (mean +/standard deviation: length 184 +/- 8, weight 74 +/- 8 kg, age 29 +/- 5 years, VO2max 64 +/- 3 ml/kg/min), Wmax 373 +/-42 W).

Before starting the study, volunteers had a medical examination and anthropometric measurements (body mass, height). Subsequently, an aerobic capacity test was performed with a graded exercise test on the bike until exhaustion, determining maximum oxygen uptake, maximum power and maximum heart rate.

Volunteers performed the standard graded exercise test to volitional exhaustion in a medicalized Unit to determine maximal workload (Wmax) and maximal oxygen uptake (VO2max). This test was performed on an electrically braked cycle ergometer (Lode Excalibur sport).

It started at a work rate of 100 W for 5 min after which the work rate was increased every 2 min by 30 W until the volunteer could no longer maintain the required pedal frequency (>60 rpm). Heart rate (HR) was recorded continuously by a radiotelemetry heart rate monitor (Polar Vantage NV, Kempele, Finland). Oxygen consumption and carbon dioxide production was monitored continuously throughout the test using an online automated gas analysis system.

Subjects were asked to keep a dietary and training log 2 days before the first experimental trial ― for subsequent trials, they were asked to repeat this activity and diet pattern. In particular, subjects were asked to refrain from strenuous training and alcohol the day before each experimental trial. The training and diet log was discussed after each experiment to clear uncertainties.

### Study Design

This was a double blind, randomized, single center, clinical trial (scheme).

Each subject completed three experimental sessions on separate days:
- **Drink A: a maltodextrin+glucose drink**
- **Drink B: a maltodextrin+fructose drink**
- **Drink C: a maltodextrin+galactose drink**

The drinks were iso-energetic and iso-osmotic.

**Scheme. Trial design**

| *Study plan:* | **Session 0** | **Session 1** | **Session 2** | **Session 3** |
|---|---|---|---|---|
| History/Subjects data | × | | | |
| Clinical examination | × | | | |
| Informed consent | × | | | |
| Schedule the trials | × | | | |
| Graded bicycle VO₂max test | × | | | |
| Gycogen depleting exercise | | × | × | × |
| Magnetic resonance spectroscopy/imaging | | × | × | × |
| Blood sampling | | × | × | × |
| Carbohydrate treatment | | RANDOM | RANDOM | RANDOM |
| Adverse events | × | × | × | × |

### Products

Products were obtained from different suppliers: maltodextrin (Glucidex DE12, Roquette Freres, F-62080 Lestrem, France), crystalline fructose (Fruitose S, Galam Ltd via Sugro AG, CH-4002 Basel, Switzerland), D-(+)-galactose (Aldrich Fluka Chemie GmbH, CH-9470 Buchs, Switzerland), D-(+)-glucose (Roferose ST, Roquette Frères, F-62080, Lestrem, France) and were mixed by Nestle.

Analysis of carbohydrates in the final blends by NUK Quality Assurance Laboratory indicated maltodextrin:monosaccharide ratios of 1.9:1 (A), 2.0:1 (B) and 1.9 :1 (C) with the respective monosaccharide.

The drinks were prepared in powder form and conditioned in individual sachets. The sachets contained 45g of carbohydrate powder and were labelled as A, B or C. They were provided in a blinded fashion, i.e. the volunteers as well as the staff conducting the study were blinded for the type of beverage.

Sachets were dissolved in an appropriate volume of water (300 ml per sachets) immediately before usage throughout an experimental trial. Following dissolution in water, the products were administered orally.

### Intake

The average rate of carbohydrate and fluid intake during the 6h-recovery period was 1.25 g/min and 600 ml/h overall, respectively. Subjects received 600 ml immediately after the basal MRS measurement followed by 300 ml every 45 min, until the start of the next MRS measurement. The total amount of carbohydrate and fluid provided during each experimental trial was 450 g dissolved in 3 1 water and a drop of citron juice according to the taste of the volunteer.

Randomization of subjects to treatments and period was performed by a program for randomization.

### Test sessions

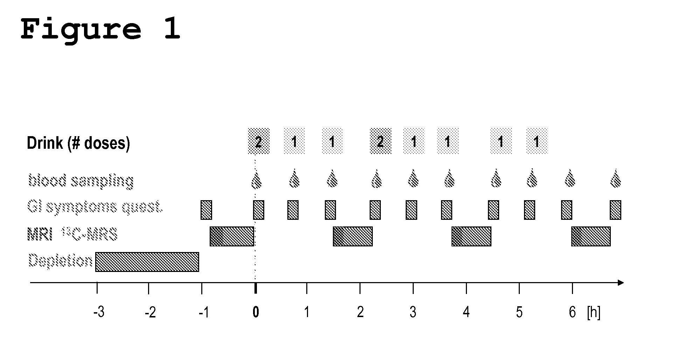

**Figure** 1 shows the timing of a single day (i.e. one specific drink). With the end of the basal MR examination, the timing starts with t=0. Since the duration of the depletion period until exhaustion was individual, the timing until t=0 was variable. An EXCEL sheet was prepared such that the starting time could be entered and a sheet was printed with the exact timing of the very day. On average, deviations from the target timing were better than 5 minutes.

### Glycogen Depleting Exercise and Carbohydrate Feeding

Volunteers were asked to attend the laboratory in the morning after an overnight fast to undergo a cycling exercise protocol to deplete their glycogen stores.

The protocol started with a 10 min warm up at 50% of maximal power output. Thereafter, participants were asked to cycle for 2 min periods alternating between 90 and 50% Wmax, until they were no longer able to complete 2 min at 90% Wmax. At this point the high exercise block was reduced to 80% Wmax, and the alternating process continued until this intensity could no longer be maintained, after which the high intensity block was decreased to 70% Wmax. When the volunteers were no longer able to switch between 70% and 50% in 2 minutes intervals, they were motivated to maintain levels of workload that were still tolerated (typically 50%) until complete exhaustion. Water was provided ad libitum during the exercise protocol. This protocol is an established method for inducing muscle glycogen depletion in trained cyclists (Kuipers 1987). After finishing the protocol, participants were allowed to take a brief shower and asked to return to the laboratory within approx. 30 min after cessation of the exercise protocol.

While supine, a Teflon catheter (Venflon 20G) was inserted into an antecubital vein in the arm in order to allow for repeated blood sampling during the experimental protocol. Next, the volunteer underwent a basal MR-measurement, followed by the extraction of a resting blood sample (7 ml). Volunteers were then asked to consume the first bolus of test drink (t=0 min, 600 ml, 2 sachets) and received subsequent drinks roughly every 45 minutes (Figure 1). Additional blood samples were collected at intervals as shown in Figure 1 for the measurement of plasma glucose and insulin concentrations. The total amount of blood taken was approximately 80 ml per trial.

The ingested drink ensured that either MD+glucose, or MD+fructose or MD+galactose were ingested at an average rate of 1.5 g/min or 90 g per hour initially. The order of the trials was been randomized as described above.

### Magnetic Resonance Spectroscopy Measurements

The study made use of Natural-abundance 13C NMR spectroscopy. NMR is a non-invasive technique that enables in vivo assessments of muscle and/or liver glycogen concentrations.

13C NMR has been developed and used to obtain information about human glycogen metabolism with diet and exercise. Since NMR is non-invasive, more data points are available over a specified time course, dramatically improving the time resolution in comparison with chemical analysis, which necessitates a surgical sampling of human muscle. This improved time resolution enables the documentation of subtleties of glycogen re-synthesis following severe glycogen depletion that were not previously observed.

Muscle and liver glycogen concentrations have been tracked in several different human populations under various conditions.

The clinical MR scanner is able to observe C-signals in liver. In addition technical equipment is used to acquire ¹³C-MR spectra of the human liver. It is important that signal noise is kept low, to allow a low signal-to-noise-ratio and the absolute quantification [mM] in order to obtain useful results.

MR images and ¹H decoupled natural abundance ¹³C-MR spectra were recorded on a standard 1.5 Tesla GE SIGNA system (General Electric Milwaukee WI) equipped with a home-built decoupler console for decoupling and Nuclear Overhauser effect build-up. Experimental details of the measurement are described in (Boesch 2001; Fluck 2003).

The body coil was used to obtain MR images for positioning while a double-tuned flexible surface coil (Medical Advance, Milwaukee WI) was used for ¹³C signal excitation and reception.

In order to position the volunteers reproducibly within a few millimetres, a thorough positioning procedure was used that has been implemented for a foregoing study (Boesch 2001).

Quantification of the glycogen signal was obtained in two steps. Since volunteers served as their own control in the crossover-design of the study, careful positioning of the surface coil helped to obtain the same sensitivity distribution in all sessions for one particular volunteer.

In order to translate institutional units (which are comparable within one volunteer, however, need an individual correction factor depending on the distance between coil and liver) into absolute units [mM], a 95 mM glycogen solution in a liver-shaped phantom was measured in the same distance from the coil centre as in the volunteers. These distances have been determined by MRI in volunteers and phantom.

### Questionnaire on gastrointestinal side-effects

Questions evaluating perceptions of the subjects upon ingesting such large volumes of carbohydrate drinks wereasked at intervals in the course of recovery using scales from 1 (not at all) to 10 (very, very much).

### Results

The exercise to exhaustion lasted (mean +/- SD) 118 +/- 8 min and was realized at 59 +/- 3% Wmax (average of alternated intensities). Total work was 1'564 +/- 281 kJ.

### Glycogen Concentrations

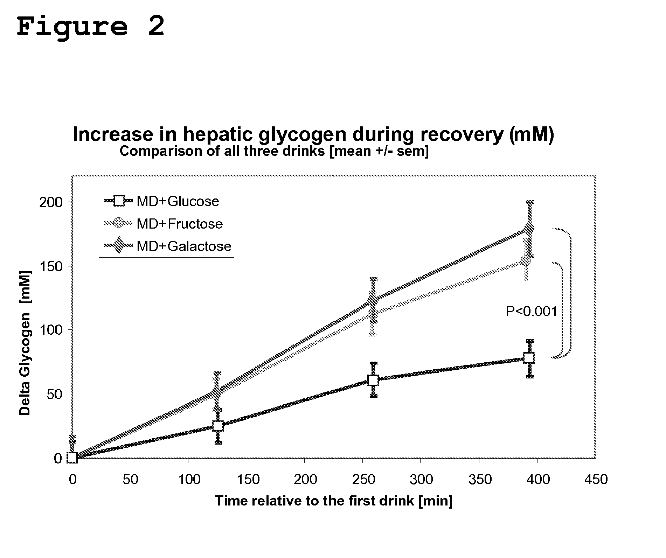

The comparison of the glycogen levels for all three drinks during recovery shows that Glucose has a lower slope of glycogen replenishment than Fructose and Galactose.
See Figure 2.

In Figure 2 it is clear that the replenishment with Glucose is during all periods lower than with the other drinks. It is also noteworthy that all drinks lead the highest relative replenishment rate during the second period (2h to 4h30 after the 1^{st} drink).

Overall, mean 6-h replenishment rates for MD+fructose (0.39 mM/min, P<0.001) and MD+galactose (0.45 mM/min, P<0.001) were twice faster as compared to MD+glucose (0.20 mM/min). MD+galactose and MD+fructose were not significantly different from each other (P=0.664).

It is known from the published literature that amounts of glucose (or glucose-yielding carbohydrates) similar to those fed in this study will result in a net storage of glycogen in muscle during a similar period. The blending of glucose-yielding maltodextrin with either fructose or galactose is uniquely shown here to be more effective than the reference for replenishing glycogen in the liver. Therefore, using one of the above blends - or combination of them - is an ideal complementary technique to other acknowledged muscle loading strategies.

### Questionnaire

### Side-effects

Beside a natural need to urinate in the course of the recovery period, average scores related to the gastric and intestinal side-effects never exceeded 1.3, which is interpreted as "very weak" or essentially no side effects.

## Claims

1. Use of a food product or beverage comprising a combination of carbohydrates for replenishment of shortage in glycogen stored in the liver.

2. Use according to claim 1, wherein the combination of carbohydrates comprises
- glucose and/or glucose delivering carbohydrates, and
- fructose and/or fructose delivering carbohydrates.

3. Use according to claim 2, wherein there is a ratio of 4:1 to 1:1 between
1) glucose and glucose delivering carbohydrates and
2) fructose and fructose delivering carbohydrates.

4. Use according to claims 2 and 3, wherein the combination in addition comprises
galactose and/or galactose delivering carbohydrates.

5. Use according to claim 1, wherein the combination of carbohydrates comprises
- glucose and/or glucose delivering carbohydrates, and
- galactose and/or galactose delivering carbohydrates.

6. Use according to claim 5, wherein there is a ratio of 4:1 to 1:1 between
1) glucose and glucose delivering carbohydrates and
2) galactose and galactose delivering carbohydrates.

7. Use according to any of the preceding claims, wherein the food product or beverage is used for increasing the speed of replenishment of shortage in glycogen stored in the liver.

8. Use according to any of the preceding claims, wherein the use is in post-exercise recovery for replenishment of liver glycogen storage.

9. Use according to any of the preceding claims, wherein the use is in post-sleep recovery for replenishment of liver glycogen storage.

10. Use according to any of the preceding claims, wherein the fructose is below half of the carbohydrates being fed in grams.

11. Use according to any of the preceding claims, wherein the glucose delivering is Maltodextrin.

12. Use according to any of the preceding claims wherein the food product or beverage is used in an amount that corresponds to an ingestion of 60 to 90 grams of carbohydrate per hour, preferable from 70 to 90 grams per hour.

13. Use according to any of the preceding claims wherein the food product or beverage has an energy density of 1450 to 1970 kj/100 gram based on dry matter.

14. A post exercise food product or beverage for replenishment of shortage in glycogen stored in the liver comprising a combination of carbohydrates comprising
- glucose and/or glucose delivering carbohydrates, and
- fructose and/or fructose delivering carbohydrates,
in a ratio of 4:1 to 1:1.

15. A post exercise food product or beverage according to claim 14, wherein the combination in addition comprises
galactose and/or galactose delivering carbohydrates.

16. A post exercise food product or beverage for replenishment of shortage in glycogen stored in the liver comprising a combination of carbohydrates comprising
- glucose and/or glucose delivering carbohydrates, and
- galactose and/or galactose delivering carbohydrates. in a ratio of 4:1 to 1:1.
